# EUROPEAN PATENT APPLICATION

(11) **EP 3 808 742 A1**
(43) Date of publication of application: **21.04.2021**
(21) Application number: 19203592.1
(22) Date of filing: 16.10.2019
(51) Int. Cl.: C07D 403/12, A61K 31/497, A61P 35/00, A61P 25/00, A61P 31/00

(54) **POLYMORPH OF SELINEXOR**

(71) Applicant: Sandoz AG, 4056 Basel (CH)
(72) Inventor: NOLWENN, Martin, 6250 Kundl (AT)
(74) Representative: Kluschanzoff, Harald

(57) **Abstract**

The present invention relates to a polymorph of selinexor and to a process for its preparation. Furthermore, the invention relates to a pharmaceutical composition comprising the polymorph of selinexor of the present invention, preferably in a predetermined and/or effective amount, and at least one pharmaceutically acceptable excipient. The pharmaceutical composition of the present invention can be used as a medicament, in particular for the treatment and/or prophylaxis of cancers such as relapsed or refractory multiple myeloma (RRMM).

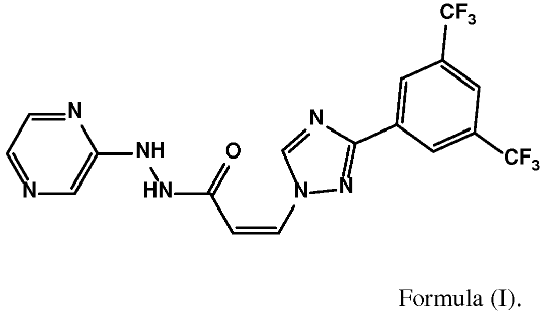

## Description

### FIELD OF THE INVENTION

The present invention relates to a polymorph of selinexor and to a process for its preparation. Furthermore, the invention relates to a pharmaceutical composition comprising the polymorph of selinexor of the present invention, preferably in a predetermined and/or effective amount, and at least one pharmaceutically acceptable excipient. The pharmaceutical composition of the present invention can be used as a medicament, in particular for the treatment and/or prophylaxis of cancers such as relapsed or refractory multiple myeloma (RRMM).

### BACKGROUND OF THE INVENTION

Selinexor is an orally available nuclear export inhibitor, which reversibly inhibits nuclear export of tumor suppressor proteins (TSPs), growth regulators and mRNAs of oncogenic proteins by blocking exportin 1 (XPO1). In combination with low-dosed dexamethasone it is indicated for the treatment of adult patients with relapsed or refractory multiple myeloma (RRMM). Selinexor is approved in the form of 20 mg tablets (Xpovio®). The chemical name of selinexor is (2Z)-3-{3-[3,5-bis(trifluoromethyl)phenyl]-1*H*-1,2,4-triazol-1-yl}-*N*'-(pyrazin-2-yl)prop-2-enehydrazide and it can be represented by the following chemical structure according to Formula (I)

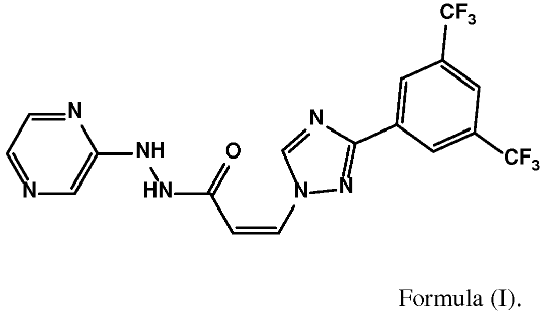

Selinexor is disclosed in WO 2013/019548 A1. Example 2 describes a process for producing selinexor, no details regarding solid-state properties of the obtained material are provided, though.

According to the teaching of WO 2016/025904 A1 a comprehensive polymorphism assessment revealed three anhydrous polymorphs designated as Form A, Form B and Form C, with Form A being the thermodynamically most stable form.

WO 2017/118940 A1 discloses various crystalline forms of selinexor, which have been characterized by powder X-ray diffraction only. Form alpha of said application corresponds to Form A of WO 2016/025904 A1.

WO 2018/129227 A1 discloses various crystalline forms of selinexor designated as Forms T1-T17 including hydrates, solvates or mixed solvates/hydrates. Only one anhydrous crystalline form designated as Form T10 is disclosed in said application.

Different solid state forms of an active pharmaceutical ingredient often possess different properties. Differences in physicochemical properties of solid forms can play a crucial role for the improvement of pharmaceutical compositions, for example, pharmaceutical formulations with improved dissolution profile and bioavailability or with improved stability or shelf-life can become accessible due to an improved solid state form of an active pharmaceutical ingredient. Also processing or handling of the active pharmaceutical ingredient during the formulation process may be improved. New solid state forms of an active pharmaceutical ingredient can thus have desirable processing properties. They can be easier to handle, better suited for storage, and/or allow for better purification, compared to previously known solid forms.

The thermodynamically most stable polymorph of an active pharmaceutical ingredient is often used for the preparation of a drug product, since phase transitions during pharmaceutical standard processes like compaction, milling and granulation can be reduced to the extent possible. However, a huge drawback connected with the thermodynamically most stable form of a drug substance is the fact, that it is the least soluble form, which translates into decreased bioavailability. This is especially critical for low soluble compounds like selinexor which is classified as a BCS class 2 substance with low solubility and high permeability. Therefore, using a metastable polymorph of a drug substance is sometimes desirable on account of its special properties, in particular higher solubility and hence bioavailability. However, it is important to avoid any possible solid form transitions, which may occur for a metastable polymorph during pharmaceutical processing or storage, since this can have a huge impact on the safety and efficacy of a drug product. Therefore, not every metastable polymorph of an active pharmaceutical ingredient can be used for the production of a pharmaceutical composition.

For example, the metastable polymorphs B, C and T10 suffer from poor stability with a high transformation tendency towards Form A. In addition, Form T10 suffers from poor crystallinity (see Figure 10 of WO 2018/129227 A1).

Aim of the present invention is therefore to address the above described issue connected with the low solubility of selinexor Form A and the low stability of Forms B, C and T10. In particular, the objective of the present invention is to provide an improved polymorph of selinexor, which shows high solubility and dissolution rates as well as good wettability and at the same time is kinetically stable e.g. does not undergo phase transformations during standard pharmaceutical processing and storage.

### SUMMARY OF THE INVENTION

The present invention solves the above mentioned problems by providing a metastable polymorph of selinexor, which possesses high kinetic stability and higher solubility compared to the thermodynamically stable Form A. Hence, the crystalline form of selinexor of the present invention combines the advantageous properties of high physical stability and high solubility and is therefore the preferred solid form of selinexor for the preparation of a pharmaceutical composition.

### Abbreviations

- PXRD: powder X-ray diffractogram
- RT: room temperature
- RH: relative humidity
- w-%: weight percent

### Definitions

In the context of the present invention the following definitions have the indicated meaning, unless explicitly stated otherwise:
As used herein the term "room temperature" refers to a temperature in the range of from 20 to 30 °C.

As used herein, the term "measured at a temperature in the range of from 20 to 30 °C" refers to a measurement under standard conditions. Typically, standard conditions mean a temperature in the range of from 20 to 30 °C, i.e. at room temperature. Standard conditions can mean a temperature of about 22 °C. Typically, standard conditions can additionally mean a measurement under 20-80% RH, preferably 30-70% RH, more preferably 40-60% RH and most preferably 50% RH.

The term "Form A" as used herein, when talking about a solid-state form of selinexor refers to the anhydrous non-solvated crystalline form of selinexor, which is disclosed in WO 2016/025904 A1. Form A of selinexor can for example be characterized by having a powder X-ray diffractogram comprising reflections at 2-Theta angles (4.4 ± 0.2)°, (12.4 ± 0.2)°, (13.1 ± 0.2)°, (19.9 ± 0.2)° and (21.3 ± 0.2)°, when measured at a temperature in the range of from 20 to 30 °C with Cu-Kalpha_{1,2} radiation having a wavelength of 0.15419 nm.

The term "reflection" with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see *"*Fundamentals of Powder Diffraction and Structural Characterization of Materials" by Vitalij K Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-Theta values is in the range of ± 0.2° 2-Theta, preferably in the range of ± 0.1° 2-Theta. Thus, a reflection that usually appears at 8.7° 2-Theta for example can appear between 8.5 and 8.9° 2-Theta, preferably between 8.6 and 8.8° 2-Theta on most X-ray diffractometers under standard conditions. Furthermore, one skilled in the art will appreciate that relative reflection intensities will show inter-apparatus variability as well as variability due to degree of crystallinity, preferred orientation, particle size, sample preparation and other factors known to those skilled in the art and should be taken as qualitative measure only.

The anhydrous and non-solvated crystalline form of selinexor of the present invention may be referred to herein as being characterized by graphical data "as shown in" a figure. Such data include, for example, powder X-ray diffraction. The person skilled in the art understands that factors such as variations in instrument type, response and variations in sample directionality, sample concentration and sample purity may lead to small variations for such data when presented in graphical form, for example variations relating to the exact reflection positions and intensities. However, a comparison of the graphical data in the figures herein with the graphical data generated for another or an unknown solid-state form and the confirmation that two sets of graphical data relate to the same crystal form is well within the knowledge of a person skilled in the art.

The terms "solid form" or "solid-state form" as used herein interchangeably refer to any crystalline and/or amorphous phase of a compound.

As used herein, the term "amorphous" refers to a solid form of a compound that is not crystalline. An amorphous compound possesses no long-range order and does not display a definitive X-ray diffraction pattern with reflections.

As used herein the term "polymorph" refers to crystalline forms having the same chemical composition but different spatial arrangements of the molecules, atoms, and/or ions forming the crystal.

The term "hydrate" as used herein, refers to a crystalline solid where either water is cooperated in or accommodated by the crystal structure e.g. is part of the crystal structure or entrapped into the crystal (water inclusions). Thereby, water can be present in a stoichiometric or non-stoichiometric amount. When water is present in stoichiometric amount, the hydrate may be referred to by adding Greek numeral prefixes. For example, a hydrate may be referred to as a *hemi*hydrate or as a *mono*hydrate depending on the water/compound stoichiometry. The water content can be measured, for example, by Karl-Fischer-Coulometry.

The term "solvate" as used herein, refers to a crystalline solid where either one or more organic solvent(s) is/are cooperated in or accommodated by the crystal structure e.g. is/are part of the crystal structure or entrapped into the crystal (solvent inclusions). Thereby, the one or more organic solvent(s) can be present in a stoichiometric or non-stoichiometric amount. When the one or more organic solvent(s) is/are present in stoichiometric amount(s), the solvate may be referred to by adding Greek numeral prefixes. For example, a solvate may be referred to as a *hemi*solvate or as a *mono*solvate depending on the solvent(s)/compound stoichiometry. The solvent content can be measured, for example, by GC, NMR, SXRD and/or TGA/MS.

The terms "anhydrous" or "anhydrate" as used herein refer to a crystalline solid where no water is cooperated in or accommodated by the crystal structure. Anhydrous forms may still contain residual water, which is not part of the crystal structure but may be adsorbed on the surface or absorbed in disordered regions of the crystal. Typically, an anhydrous form does not contain more than 2.0 w-%, preferably not more than 1.0 w-%, more preferably not more than 0.5 w- % of water, based on the weight of the crystalline form.

The term "non-solvated" as used herein, when talking about a crystalline solid indicates that no organic solvent is cooperated in or accommodated by the crystal structure. Non-solvated forms may still contain residual organic solvents, which are not part of the crystal structure but may be adsorbed on the surface or absorbed in disordered regions of the crystal. Typically, a non-solvated form does not contain more than 2.0 w-%, preferably not more than 1.0 w-%, more preferably not more than 0.5 w-% of organic solvents, based on the weight of the crystalline form.

A "predetermined amount" as used herein with regard to selinexor refers to the initial amount of selinexor used for the preparation of a pharmaceutical composition having a desired dosage strength of selinexor.

The term "effective amount" as used herein with regard to selinexor encompasses an amount of selinexor, which produces the desired therapeutic and/or prophylactic effect.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

The term "pharmaceutically acceptable excipient" as used herein refers to substances, which do not show a significant pharmacological activity at the given dose and that are added to a pharmaceutical composition in addition to the active pharmaceutical ingredient. Excipients may take the function of vehicle, diluent, release agent, disintegrating agent, dissolution modifying agent, absorption enhancer, stabilizer or a manufacturing aid among others. Excipients may include fillers (diluents), binders, disintegrants, lubricants and glidants.

The term "filler" as used herein refers to substances that are used to dilute the active pharmaceutical ingredient prior to delivery. Fillers can also serve as stabilizers.

As used herein the term "binder" refers to substances which bind the active pharmaceutical ingredient and pharmaceutically acceptable excipient together to maintain cohesive and discrete portions.

The terms "disintegrant" or "disintegrating agent" as used herein refer to substances which, upon addition to a solid pharmaceutical composition, facilitate its break-up or disintegration after administration and permit the release of the active pharmaceutical ingredient as efficiently as possible to allow for its rapid dissolution.

The term "lubricant" as used herein refers to substances which are added to a powder blend to prevent the compacted powder mass from sticking to the equipment during tableting or encapsulation process. They aid the ejection of the tablet from the dies and can improve powder flow.

The term "glidant" as used herein refers to substances which are used for tablet and capsule formulations in order to improve flow properties during tablet compression and to produce an anti-caking effect.

### BRIEF DESCRIPTION OF THE FIGURES

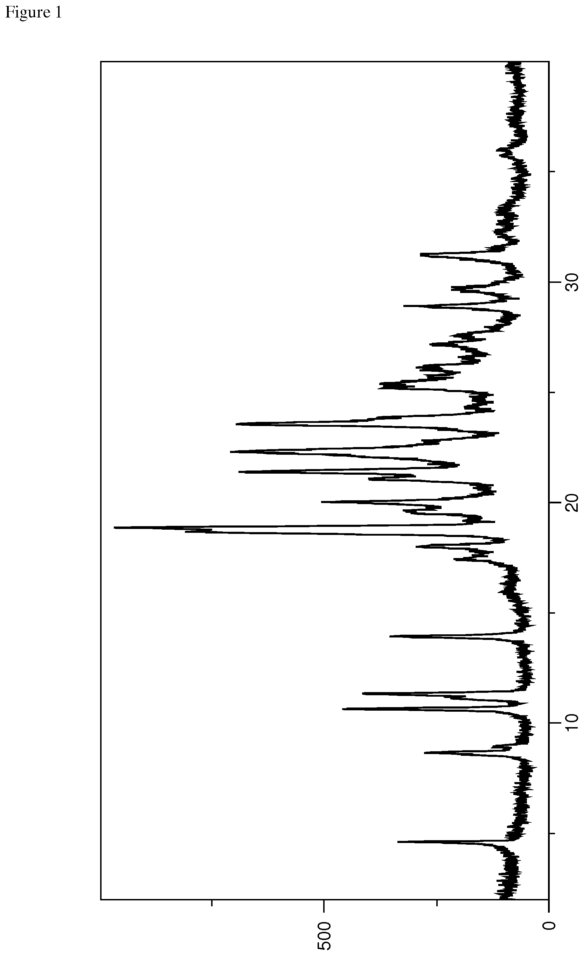

**Figure 1****:** illustrates a representative PXRD of the crystalline form of selinexor of the present invention. The x-axis shows the scattering angle in °2-Theta, the y-axis shows the intensity of the scattered X-ray beam in counts of detected photons.

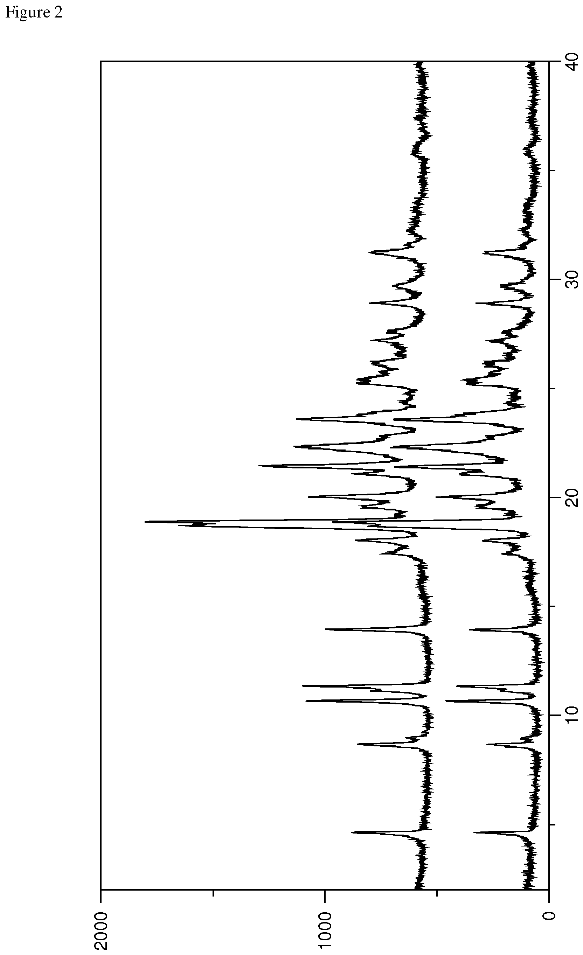

**Figure 2****:** illustrates a comparison of the PXRDs of the crystalline form of selinexor of the present invention before (bottom) and after (top) open storage at an atmosphere of 40°C / 75% RH. The x-axis shows the scattering angle in °2-Theta. The powder X-ray diffractogram of the stressed sample was shifted along the y-axis to separate the diffractograms for clarity. The y-axis is therefore arbitrary and was not labeled.

### DETAILED DESCRIPTION OF THE INVENTION

The present invention provides an anhydrous and non-solvated metastable polymorph of selinexor.

Despite being a metastable polymorph, the crystalline form of the present invention, in contrast to the already known metastable polymorphs Form B, C and T10, possesses high physical stability, e.g. it is stable against temperature, moisture and/or mechanical stress (see Example 3 herein). The fact that the crystalline form of the present invention is metastable related to the already known Form A implies that the crystalline form of the present invention shows higher solubility, a fact which translates in higher bioavailability of a low soluble drug like selinexor. Hence, the crystalline form of the present invention combines the advantageous properties of high physical stability and high solubility and is therefore the ideal solid form of selinexor for the preparation of an improved pharmaceutical composition e.g. a pharmaceutical composition with increased bioavailability, which is safe and effective during its whole shelf-life.

The crystalline form of selinexor of the present invention may be characterized by analytical methods well known in the field of the pharmaceutical industry for characterizing solids. Such methods comprise but are not limited to PXRD and FTIR. It may be characterized by one of the aforementioned analytical methods or by combining them. In particular, the crystalline form of selinexor of the present invention may be characterized by any one of the following embodiments or by combining two or more of the following embodiments.

In one embodiment the invention relates to an anhydrous and non-solvated crystalline form of selinexor characterized by having a PXRD comprising reflections at 2-Theta angles of:
(4.6 ± 0.2)°, (8.7 ± 0.2)° and (13.9 ± 0.2)°; or
(4.6 ± 0.2)°, (8.7 ± 0.2)°, (10.6 ± 0.2)° and (13.9 ± 0.2)°; or
(4.6 ± 0.2)°, (8.7 ± 0.2)°, (10.6 ± 0.2)°, (11.3 ± 0.2)° and (13.9 ± 0.2)°; or
(4.6 ± 0.2)°, (8.7 ± 0.2)°, (10.6 ± 0.2)°, (11.3 ± 0.2)°, (13.9 ± 0.2)° and (18.9 ± 0.2)°; or
(4.6 ± 0.2)°, (8.7 ± 0.2)°, (10.6 ± 0.2)°, (11.3 ± 0.2)°, (13.9 ± 0.2)°, (18.9 ± 0.2)° and (23.6 ± 0.2)°; or
(4.6 ± 0.2)°, (8.7± 0.2)°, (10.6 ± 0.2)°, (11.3 ± 0.2)°, (13.9 ± 0.2)°, (18.9 ± 0.2)°, (22.3 ± 0.2)° and (23.6 ± 0.2)°; or
(4.6 ± 0.2)°, (8.7 ± 0.2)°, (10.6 ± 0.2)°, (11.3 ± 0.2)°, (13.9 ± 0.2)°, (18.9 ± 0.2)°, (21.4 ± 0.2)°, (22.3 ± 0.2)° and (23.6 ± 0.2)°; or
(4.6 ± 0.2)°, (8.7 ± 0.2)°, (10.6 ± 0.2)°, (11.3 ± 0.2)°, (13.9 ± 0.2)°, (18.7 ± 0.2)°, (18.9 ± 0.2)°, (21.4 ± 0.2)°, (22.3 ± 0.2)° and (23.6 ± 0.2)°,
when measured at a temperature in the range of from 20 to 30 °C with Cu-Kalpha_{1,2} radiation having a wavelength of 0.15419 nm.

In another embodiment, the invention relates to an anhydrous and non-solvated crystalline form of selinexor characterized by having a PXRD comprising reflections at 2-Theta angles of (4.6 ± 0.2)°, (10.6 ± 0.2)°, (11.3 ± 0.2)°, (13.9 ± 0.2)°, (18.7 ± 0.2)°, (18.9 ± 0.2)°, (20.0 ± 0.2)°, (21.4 ± 0.2)°, (22.3 ± 0.2)° and (23.6 ± 0.2)°, when measured at a temperature in the range of from 20 to 30 °C with Cu-Kalpha_{1,2} radiation having a wavelength of 0.15419 nm.

In a further embodiment, the present invention relates to an anhydrous and non-solvated crystalline form of selinexor characterized by having a PXRD comprising reflections at 2-Theta angles of:
(4.6 ± 0.1)°, (8.7 ± 0.1)° and (13.9 ± 0.1)°; or
(4.6 ± 0.1)°, (8.7 ± 0.1)°, (10.6 ± 0.1)° and (13.9 ± 0.1)°; or
(4.6 ± 0.1)°, (8.7 ± 0.1)°, (10.6 ± 0.1)°, (11.3 ± 0.1)° and (13.9 ± 0.1)°; or
(4.6 ± 0.1)°, (8.7 ± 0.1)°, (10.6 ± 0.1)°, (11.3 ± 0.1)°, (13.9 ± 0.1)° and (18.9 ± 0.1)°; or
(4.6 ± 0.1)°, (8.7 ± 0.1)°, (10.6 ± 0.1)°, (11.3 ± 0.1)°, (13.9 ± 0.1)°, (18.9 ± 0.1)° and (23.6 ± 0.1)°; or
(4.6 ± 0.1)°, (8.7± 0.1)°, (10.6 ± 0.1)°, (11.3 ± 0.1)°, (13.9 ± 0.1)°, (18.9 ± 0.1)°, (22.3 ± 0.1)° and (23.6 ± 0.1)°; or
(4.6 ± 0.1)°, (8.7 ± 0.1)°, (10.6 ± 0.1)°, (11.3 ± 0.1)°, (13.9 ± 0.1)°, (18.9 ± 0.1)°, (21.4 ± 0.1)°, (22.3 ± 0.1)° and (23.6 ± 0.1)°; or
(4.6 ± 0.1)°, (8.7 ± 0.1)°, (10.6 ± 0.1)°, (11.3 ± 0.1)°, (13.9 ± 0.1)°, (18.7 ± 0.1)°, (18.9 ± 0.1)°, (21.4 ± 0.1)°, (22.3 ± 0.1)° and (23.6 ± 0.1)°,
when measured at a temperature in the range of from 20 to 30 °C with Cu-Kalpha_{1,2} radiation having a wavelength of 0.15419 nm.

In yet another embodiment, the invention relates to an anhydrous and non-solvated crystalline form of selinexor characterized by having a PXRD comprising reflections at 2-Theta angles of (4.6 ± 0.1)°, (10.6 ± 0.1)°, (11.3 ± 0.1)°, (13.9 ± 0.1)°, (18.7 ± 0.1)°, (18.9 ± 0.1)°, (20.0 ± 0.1)°, (21.4 ± 0.1)°, (22.3 ± 0.1)° and (23.6 ± 0.1)°, when measured at a temperature in the range of from 20 to 30 °C with Cu-Kalpha_{1,2} radiation having a wavelength of 0.15419 nm.

The PXRD of the crystalline form of the present invention can be readily distinguished from the PXRD of already known anhydrous and non-solvated polymorphs of selinexor such as Form A of WO 2016/025904 A1. For example, the crystalline form of the present invention shows besides others a characteristic reflections at (8.7 ± 0.2)° 2-Theta, where Form A shows no reflection. On the other hand Form A shows besides others characteristic reflections at (12.4 ± 0.2)° and (13.1 ± 0.2)° 2-Theta, whereas the crystalline form of the present invention shows no reflections in the same ranges.

Hence, in another embodiment the present invention relates to an anhydrous and non-solvated crystalline form of selinexor characterized by having a PXRD as defined in any one of the above described embodiments, and comprising no reflections at 2-Theta angles of (12.4 ± 0.2)° and/or (13.1 ± 0.2)°, when measured at a temperature in the range of from 20 to 30 °C with Cu-Kalpha_{1,2} radiation having a wavelength of 0.15419 nm.

In yet another embodiment, the present invention relates to an anhydrous and non-solvated crystalline form of selinexor characterized by having a PXRD essentially the same as shown in Figure 1 of the present invention, when measured at a temperature in the range of from 20 to 30 °C with Cu-Kalpha_{1,2} radiation having a wavelength of 0.15419 nm.

In another aspect, the present invention relates to a composition comprising the anhydrous and non-solvated crystalline form of selinexor of the present invention as defined in any one of the above described embodiments, said composition being essentially free of any other solid-state form of selinexor. For example, a composition comprising the crystalline form of selinexor of the present invention comprises at most 20 w-%, preferably at most 10 w-%, more preferably at most 5, 4, 3, 2 or 1 w-% of any other solid-state form of selinexor, based on the weight of the composition. Preferably, the any other solid-state form of selinexor is form A of WO 2016/025904 A1. Form A of selinexor exhibits a PXRD comprising amongst others characteristic reflections at 2-Theta angles of (12.4 ± 0.2)° and (13.1 ± 0.2)°, when measured at a temperature in the range of from 20 to 30 °C with Cu-Kalpha_{1,2} radiation having a wavelength of 0.15419 nm. Therefore, the absence of reflections at 2-Theta angles of (12.4 ± 0.2)° and (13.1 ± 0.2)° in the PXRD confirms the absence of form A of selinexor in the composition.

Hence, in a preferred embodiment, the present invention relates to a composition comprising the anhydrous and non-solvated crystalline form of selinexor of the present invention as defined in any one of the above described embodiments, said composition having a PXRD comprising no reflections at 2-Theta angles of (12.4 ± 0.2)° and (13.1 ± 0.2)°, when measured at a temperature in the range of from 20 to 30 °C with Cu-Kalpha_{1,2} radiation having a wavelength of 0.15419 nm.

In another embodiment, the invention relates to a composition comprising at least 90 w-%, including at least 90, 91, 92, 93, 94, 95, 96, 97, 98 and 99 w-%, and also including equal to about 100 w-% of the anhydrous and non-solvated crystalline form of selinexor of the present invention as defined in any one of the above described embodiments, based on the total weight of the composition. The remaining material may comprise other solid-state form(s) of selinexor, and/or reaction impurities and/or processing impurities arising from the preparation of the composition.

In another aspect, the present invention relates to a process for the preparation of the anhydrous and non-solvated crystalline form of selinexor of the present invention or the composition comprising the same as defined in any one of the above described aspects and their corresponding embodiments comprising:
(a) providing a solution comprising selinexor and at least one cyclic ether;
(b) optionally, adding seed crystals of the crystalline form of selinexor of the present invention;
(c) evaporating at least a part of the at least one cyclic ether provided in (a) in order to initiate crystallization;
(d) isolating at least a part of the crystals obtained in (c);
(e) drying the crystals obtained in (d);

Selinexor may be prepared according to any method known to the skilled person e.g. according to one of the procedures provided in WO 2013/019548 A1 (e.g. Example 2) or WO 2016/025904 A1 (e.g. Example 1). Since the selinexor starting material is dissolved in step (a) the solid-state of the starting material is not critical. Any solid form of selinexor can be used e.g. crystalline selinexor, amorphous selinexor or mixtures thereof. Suitable crystalline forms may for example be selected from Forms A to D of WO 2016/025904 A1 or Forms T1-T17 of WO 2018/129227 A1, which may be prepared according to the teachings provided in the descriptions of the respective patent applications. Preferably, the starting material is amorphous selinexor, which can be prepared according to the procedure described in Example 4 hereinafter. The at least one cyclic ether in step (a) of the above described process is selected from the group consisting of tetrahydrofuran, methyltetrahydrofuran, 1,4-dioxane or any mixtures thereof. Preferably, methyltetrahydrofuran or 1,4-dioxane are used. In order to dissolve selinexor in the at least one cyclic ether in step (a) the solvent is added at a temperature in the range of from 25 to 60 °C, more preferably in the range of from 40 to 50 °C, preferably at ambient pressure. The mixture may optionally be sonicated at a temperature in the range of from 25 to 60 °C to accelerate dissolution of the solid material. Optionally, the solution provided in step (a) may be filtered in order to remove any possible undissolved particles.

Optionally, seed crystals may be added in step (b) in order to promote crystallization and/or to control particle size distribution. The seed crystals may be prepared according to the same process as the one reported herein for the preparation of the crystalline form of selinexor of the present invention. In particular, the seed crystals may be prepared according to the procedures provided in Examples 1 and 2 hereinafter. Typically, the seed crystals are added in an amount in the range of from about 0.1 to 10 w-%, preferably of from about 0.5 w-% to 7.0 w-%, most preferably of from about 1.0 to 5.0 w-%, on the basis of the total amount of the starting material used in step (a).

In step (c) at least a part, preferably all of the at least one cyclic ether is evaporated. Thereby, the at least one cyclic ether is evaporated at a temperature in the range of from about 10 to 60 °C, preferably of from 20 to 40 °C. Evaporation may be performed under reduce pressure or at ambient pressure. Preferably, crystallization is initiated by slow evaporation of the at least cyclic ether at ambient conditions until crystallization occurs. The evaporation is preferably performed without mechanical agitation. In this context, the term "mechanical agitation" relates to any motion of a macroscopic constituent of the solution comprising selinexor, which is induced from outside via a device, such as shaking or stirring or sonication, relative to another macroscopic constituent of the solution. Once crystallization started, the mixture can optionally be cooled to a temperature in the range from 0 to 20 °C, preferably in the range from 5 to 15 °C and/or one or more anti-solvents can be added to the mixture to increase the process yield. Regarding the antisolvents, no specific restrictions exist, provided that the crystalline form of selinexor of the present invention is not soluble and is stable.

Once the crystalline form of selinexor of the present invention is obtained, at least a part of the crystals is isolated. The crystals may be isolated by any conventional method such as filtration, centrifugation, solvent evaporation or decantation. Optionally, in a further step the isolated crystals are washed with a suitable solvent or solvent mixture. Regarding the suitable solvent or solvent mixture, no specific restrictions exist, provided that the crystalline form of selinexor of the present invention is not soluble and is stable.

The obtained crystals may then optionally be dried. Drying may be performed at a temperature in the range of from about 20 to 80 °C, preferably in the range of from about 20 to 40 °C and most preferably drying is performed at about RT. Drying may be performed for a period in the range of from about 1 to 72 hours, preferably of from about 2 to 48 hours, more preferably of from about 4 to 24 hours and most preferably of from about 6 to 18 hours. Drying may be performed at ambient pressure and/ or under reduced pressure. Preferably, drying is performed at reduced pressure of about 100 mbar or less, more preferably of about 50 mbar or less, for example a vacuum of about 35 mbar is applied for drying.

In a further aspect the present invention relates to the use of the anhydrous and non-solvated crystalline form of selinexor of the present invention or the composition comprising the same as defined in any one of the above described aspects and their corresponding embodiments for the preparation of a pharmaceutical composition.

In yet another aspect, the present invention relates to a pharmaceutical composition comprising the anhydrous and non-solvated crystalline form of selinexor of the present invention or the composition comprising the same as defined in any one of the above described aspects and their corresponding embodiments, preferably in a predetermined and/or effective amount, and at least one pharmaceutically acceptable excipient.

Preferably, the predetermined and/or effective amount of the anhydrous and non-solvated crystalline form of selinexor of the present invention or the composition comprising the same as defined in any one of the above described aspects and their corresponding embodiments is selected from the group consisting of 20 mg, 30 mg, 40 mg, 50 mg, 60 mg, 70 mg, 80 mg, 90 mg and 100 mg, calculated as anhydrous and non-solvated selinexor. More preferably, the predetermined and/or effective amount of the anhydrous and non-solvated crystalline form of selinexor of the present invention or the composition comprising the same as defined in any one of the above described aspects and their corresponding embodiments is selected from the group consisting of 20 mg, 40 mg, 60 mg, 80 mg and 100 mg calculated as anhydrous and non-solvated selinexor. More preferably, the predetermined and/or effective amount of the anhydrous and non-solvated crystalline form of selinexor of the present invention or the composition comprising the same as defined in any one of the above described aspects and their corresponding embodiments is 20 mg, 40 mg, 60 mg and 80 mg and most preferably 20 mg calculated as anhydrous and non-solvated selinexor.

The at least one pharmaceutically acceptable excipient, which is comprised in the pharmaceutical composition of the present invention, is preferably selected from the group consisting of fillers, binders, disintegrants, lubricants, glidants and combinations thereof. Preferably, the at least one pharmaceutically acceptable excipient is selected from the group consisting of one or more fillers, a disintegrant and a lubricant. More preferably, the at least one pharmaceutically acceptable excipient is selected from the group consisting of microcrystalline cellulose, colloidal silicon dioxide, croscarmellose sodium, magnesium stearate and combinations thereof. Most preferably, all of these pharmaceutically acceptable excipients are comprised by the pharmaceutical composition of the present invention.

In a preferred embodiment, the pharmaceutical composition comprising the anhydrous and non-solvated crystalline form of selinexor of the present invention or the composition comprising the same as defined in any one of the above described aspects and their corresponding embodiments is an oral solid dosage form. More preferably, the oral solid dosage form is a tablet or a capsule, most preferably a tablet. In a particular embodiment, the pharmaceutical composition of the present invention as described above is a tablet, preferably a film-coated tablet, even more preferably an immediate-release film-coated tablet.

The pharmaceutical compositions of the present invention as defined in any one of the above described embodiments may be produced by standard manufacturing processes, which are well-known to the skilled person including e.g. blending, granulation (wet or dry granulation), tablet compression, film-coating or capsule filling and packaging.

For example, the tablet may be prepared by mixing the anhydrous and non-solvated crystalline form of selinexor of the present invention or the composition comprising the same as defined in any one of the above described aspects and their corresponding embodiments with at least one excipient such as fillers, binders, disintegrants, lubricants, glidants or combinations thereof. Optionally, a granulation step such as a dry or wet granulation step is performed before compression. The tablet cores may be additionally film-coated.

For example, the capsule may be prepared by mixing the anhydrous and non-solvated crystalline form of selinexor of the present invention or the composition comprising the same as defined in any one of the above described aspects and their corresponding embodiments with at least one excipient such as fillers, binders, disintegrants, lubricants, glidants or combinations thereof and filling the blend into a capsule. The capsule shell may be a gelatin shell or a hydroxypropylmethylcellulose (HPMC) shell.

In a further aspect, the present invention relates to the anhydrous and non-solvated crystalline form of selinexor of the present invention or the composition comprising or the pharmaceutical composition comprising the same as defined in any one of the above described aspects and their corresponding embodiments for use as a medicament.

In yet another aspect, the present invention relates to the anhydrous and non-solvated crystalline form of selinexor of the present invention or the composition or the pharmaceutical composition comprising the same as defined in any one of the above described aspects and their corresponding embodiments for use in the treatment and/or prophylaxis of cancer.

Alternatively, the invention concerns a method of treating and/or preventing cancer, said method comprising administering an effective amount of the anhydrous and non-solvated crystalline form of selinexor or the composition or the pharmaceutical composition comprising the same as defined in any one of the above described aspects and their corresponding embodiments to a patient in need of such a treatment.

In some embodiments, the cancer is selected from the group consisting of lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, caner of the penis, prostate cancer, chronic or acute leukemia, lymphocytic lymphomas, cancer of the bladder, cancer of the kidney or ureter, renal cell carcinoma, carcinoma of the renal pelvis, neoplasm of the central nervous system (CNS), primary CNS lymphoma, spinal axis tumors, brain stem glioma, or pituitary adenoma, and combinations thereof. In a preferred embodiment, the cancer is relapsed or refractory multiple myeloma (RRMM).

### EXAMPLES

The following non-limiting examples are illustrative for the disclosure and are not to be construed as to be in any way limiting for the scope of the invention.

### Example 1: Preparation of the crystalline form of selinexor of the present invention

### Method A1:

To selinexor (208.0 mg, amorphous material prepared according to the procedure described in Example 4 herein) warm 2-methyltetrahydrofuran (1.4 mL) was added. The mixture was heated at a temperature of about 50 °C followed by filtration through a syringe filter (pore size 0.45 microns). The obtained clear solution was allowed to stand in a closed test tube at room temperature without mechanical agitation. After ten days still no solid formation was observed. Therefore, the test tube was slightly opened to allow for slow solvent evaporation. The next day, a turbid solution was formed. The test tube was closed to allow for slow crystal formation. After further 24 hours, deposited crystals were collected by filtration, washed twice with 2-methyltetrahydrofuran (2 x 0.3 mL) and dried at room temperature under vacuum (about 30 mbar) for 18 hours to yield 163.0 mg of the crystalline form of selinexor of the present invention.

### Method A2:

To selinexor (140.6 mg, amorphous material prepared according to the procedure described in Example 4 herein) warm 2-methyltetrahydrofuran (1.0 mL) was added. The mixture was sonicated at a temperature of about 50 °C followed by filtration through a syringe filter (pore size 0.45 microns). About 0.3 mL of the clear filtrate was allowed to stand in a closed vial at room temperature without mechanical agitation. After one day still no solid formation was observed. Therefore, the vial was slightly opened to allow for slow solvent evaporation. After two weeks all the solvent had evaporated and the deposited crystals were dried at room temperature under vacuum (about 30 mbar) for 16 hours to yield the crystalline form of selinexor of the present invention.

### Method B:

To selinexor (141.4 mg, amorphous material prepared according to the procedure described in Example 4) warm 1,4-dioxane (1.0 mL) was added. The mixture was sonicated at a temperature of about 50 °C followed by filtration through a syringe filter (pore size 0.45 microns). About 0.3 mL of the clear filtrate was allowed to stand in a closed vial at room temperature without mechanical agitation. After one day still no solid formation was observed. Therefore, the vial was slightly opened to allow for slow solvent evaporation. After two weeks all the solvent had evaporated and the deposited crystals were dried at room temperature under vacuum (about 30 mbar) for 16 hours to yield the crystalline form of selinexor of the present invention.

### Example 2: Solid-state characterization of the crystalline form of selinexor of the present invention

### Powder X-ray diffraction

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. The diffractogram was recorded at a tube voltage of 45 kV and a tube current of 40 mA, applying a stepsize of 0.013° 2-theta with 40s per step (255 channels) in the angular range of 2° to 40° 2-Theta at ambient conditions.

A representative diffractogram of the crystalline form of selinexor of the present invention is displayed in Figure 1 herein. The corresponding reflection list is provided in Table 1 below.

**Table 1: PXRD reflection positions of the crystalline form of selinexor of the present invention in the range of from 2 to 30° 2-Theta; a typical precision of the 2-Theta values is in the range of ± 0.2° 2-Theta, preferably of ± 0.1° 2-Theta.**

| **Reflection position [° 2-Theta]** | **Reflection position [° 2-Theta]** | **Reflection position [° 2-Theta]** | **Reflection position [°2-Theta]** |
|---|---|---|---|
| 4.6 | 17.5 | 21.1 | 26.1 |
| 8.7 | 18.0 | 21.4 | 27.2 |
| 9.0 | 18.7 | 22.3 | 27.6 |
| 10.6 | 18.9 | 23.6 | 28.9 |
| 11.3 | 19.6 | 25.2 | 29.7 |
| 13.9 | 20.0 | 25.4 | |

The PXRD of the crystalline form of the present invention can be readily distinguished from the PXRDs of already known anhydrous and non-solvated crystalline forms such as Forms A, B and C of WO 2016/025904 A1 and Form T10 of WO 2018/129227 A1. For example, the crystalline form of the present invention shows characteristic reflections at (4.6 ± 0.2)° and (8.7 ± 0.2)° 2-Theta, whereas none of the above listed polymorphs show reflections at both positions. While Form C shows no reflection at either positions, Form B and Form T10 show no reflection at (4.6 ± 0.2)° and Form A shows no reflection at (8.7 ± 0.2)°. Hence, these two PXRD reflections alone are already suitable to differentiate the crystalline form of the present invention from other anhydrous and non-solvated polymorphs of selinexor.

### Example 3: Stability testing

### Stress Condition A:

The crystalline form of selinexor of the present invention was stored at ambient condition and the phase consistency analyzed periodically using powder X-ray diffraction. No solid-state transformation was seen within 35 days.

### Stress Condition B:

The crystalline form of selinexor of the present invention was subjected to accelerated stress conditions of 40 °C / 75% RH and the phase identity checked periodically with powder X-ray diffraction. Within 35 days no solid-state transformation was seen.

### Stress Condition C:

A gravimetric moisture sorption experiment was performed with the crystalline form of selinexor of the present invention using an SPSx-1µ moisture sorption analyzer (ProUmid, Ulm). The measurement cycle was started at ambient RH of 40%. The RH was then decreased to 5% in 5% steps, followed by a further decrease to 3% and to 0%. Afterwards the RH was increased from 0% to 90% in a sorption cycle and decreased to 0% in a desorption cycle in 5% steps. Finally the RH was increased to a relative humidity of 40% in 5% steps. The time per step was set to a minimum of 2 hours and a maximum of 6 hours. If an equilibrium condition with a constant mass of ± 0.01% within 1 hour was reached before the maximum time for all examined samples the sequential humidity step was applied before the maximum time of 6 hours. If no equilibrium was achieved the consecutive humidity step was applied after the maximum time of 6 hours. The temperature was 25 ± 0.1 °C. The phase identity was checked before and after the GMS experiment with powder X-ray diffraction and indicated no phase change.

### Example 4: Preparation of amorphous selinexor

To selinexor (2.80 g, crystalline Form A, e.g. prepared according to the procedure disclosed in Example 1 of WO2016/025904) warm acetone (200 mL) was added, yielding a turbid solution.

After filtration through a syringe filter (pore size 0.45 microns) a clear solution was obtained. The solvent was evaporated in a rotary evaporator at 40 °C under reduced pressure, and the remaining solid residue was dried at a temperature of about 40°C under vacuum (about 30 mbar) for 18 hours to quantitatively yield amorphous selinexor.

## Claims

1. An anhydrous and non-solvated crystalline form of (2Z)-3-{3-[3,5-bis(trifluoromethyl)phenyl]-1*H*-1,2,4-triazol-1-yl}-*N*'-(pyrazin-2-yl)prop-2-enehydrazide (selinexor) **characterized by** having a powder X-ray diffractogram comprising reflections at 2-Theta angles of (4.6 ± 0.2)°, (8.7 ± 0.2)° and (13.9 ± 0.2)°, when measured at a temperature in the range of from 20 to 30 °C with Cu-Kalpha_{1,2} radiation having a wavelength of 0.15419 nm.

2. The crystalline form of claim 1 **characterized by** having a powder X-ray diffractogram comprising additional reflections at 2-Theta angles of (10.6 ± 0.2)° and/or (11.3 ± 0.2)°, when measured at a temperature in the range of from 20 to 30 °C with Cu-Kalpha_{1,2} radiation having a wavelength of 0.15419 nm.

3. The crystalline form of claim 1 or 2 **characterized by** having a powder X-ray diffractogram comprising no reflections at 2-Theta angles of (12.4 ± 0.2)° and/or (13.1 ± 0.2)°, when measured at a temperature in the range of from 20 to 30 °C with Cu-Kalpha1,2 radiation having a wavelength of 0.15419 nm.

4. A composition comprising the crystalline form as defined in any one of the preceding claims and at most 20 w-%, 10 w-%, 5 w-%, 4 w-%, 3 w-%, 2 w-% or 1 w-% of any other solid-state form of selinexor, based on the weight of the composition.

5. The composition according to claim 4, wherein the other solid-state form of selinexor is Form A **characterized by** having a powder X-ray diffractogram comprising reflections at 2-Theta angles of (4.4 ± 0.2)°, (12.4 ± 0.2)°, (13.1 ± 0.2)°, (19.9 ± 0.2)° and (21.3 ± 0.2)°, when measured at a temperature in the range of from 20 to 30 °C with Cu-Kalpha_{1,2} radiation having a wavelength of 0.15419 nm.

6. The composition as defined in claim 4 or 5, **characterized by** having a PXRD comprising no reflections at 2-Theta angles of (12.4 ± 0.2)°and/or (13.1 ± 0.2)°, when measured at a temperature in the range of from 20 to 30 °C with Cu-Kalpha_{1,2} radiation having a wavelength of 0.15419 nm.

7. Use of the crystalline form as defined in any one of claims 1 to 3 or the composition as defined in any one of claims 4 to 6 for the preparation of a pharmaceutical composition.

8. A pharmaceutical composition comprising the crystalline form as defined in any one of claims 1 to 3 or the composition as defined in any one of claims 4 to 6 and at least one pharmaceutically acceptable excipient.

9. The pharmaceutical composition of claim 8, **characterized by** comprising 20 mg of the crystalline form as defined in any one of claims 1 to 3 or the composition as defined in any one of claims 4 to 6, calculated as anhydrous and non-solvated selinexor.

10. The pharmaceutical composition of claim 8 or 9, which is an oral solid dosage form.

11. The oral dosage form of claim 10, which is a tablet.

12. The tablet of claim 10, which is an immediate release film-coated tablet.

13. The crystalline form as defined in any one of claims 1 to 3, the composition as defined in any one of claims 4 to 6 or the pharmaceutical composition as defined in any one of claims 8 to 12 for use as a medicament.

14. The crystalline form as defined in any one of claims 1 to 3, the composition as defined in any one of claims 4 to 6 or the pharmaceutical composition as defined in any one of claims 8 to 12 for use in the treatment and/or prophylaxis of cancer.

15. The use of claim 14, wherein the cancer is relapsed or refractory multiple myeloma (RRMM).
